(19) Europäisches Patentamt
European Patent Office
Office européen des brevets

(11) Publication number: **0 211 954 B1**

(12) **EUROPEAN PATENT SPECIFICATION published in accordance with Art. 158(3) EPC**

(45) Date of publication of patent specification: **17.07.91**

(51) Int. Cl.[5]: **C07C 403/16**, A61K 7/46, A23L 1/235, A23L 1/226, C11D 3/50

(21) Application number: **85901068.8**

(22) Date of filing: **21.02.85**

(86) International application number: **PCT/JP85/00074**

(87) International publication number: **WO 85/04400 (10.10.85 85/22)**

# (54) USE OF (+/-)-CIS-GAMMA-IRONE IN PERFUMERY AND NOVEL PROCESS FOR PREPARING SAME.

(30) Priority: **03.04.84 JP 65094/84**

(43) Date of publication of application: **04.03.87 Bulletin 87/10**

(45) Publication of the grant of the patent: **17.07.91 Bulletin 91/29**

(84) Designated Contracting States: **CH DE FR GB LI NL**

(56) References cited:
**DE-A- 946 985**
**JP-A- 568 311**
**JP-A- 5 334 752**

**PATENT ABSTRACTS OF JAPAN, vol. 6, no. 231 (C-135)[1109], 17th November 1982; & JP-A-57 134 428**

**AGRIC. BIOL. CHEM., vol. 47, no. 3, 1983, pages 581-586; T. KITAHARA et al.: "The synthesis of (+-)-gamma-irones"**

(73) Proprietor: **T. HASEGAWA COMPANY, LTD.**
**No. 9, Nihonbashi-Honcho 4-chome**
**Chuo-ku Tokyo(JP)**

(72) Inventor: **KAWANOBE, Tsuneo**
**1-12-805, Wakabadai Asahi-ku, Yokohama-shi**
**Kanagawa 241(JP)**
Inventor: **KOGAMI, Kunio**
**655-11, Nougaya Machida-shi**
**Tokyo 194-01(JP)**

(74) Representative: **Kraus, Walter, Dr. et al**
**Patentanwälte Kraus, Weisert & Partner**
**Thomas-Wimmer-Ring 15**
**W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 211 954 B1

## Description

### Technical Field

This invention relates to a new use of (±)-cis-gamma-irone represented by the following formula (B)

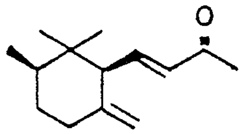

.....(B)

in the field of perfumes or flavors, and to a novel and improved process for producing the compound of formula (B).

### Background Technology

Essential oils obtained from the rhizomes of plants such as Iris pallida, Iris germanicha and Iris florentina and flowers of Cheiranthus cheriri have been also called natural orris oils and known to be perfumes having a violet-like fragrance. They are very important and expensive essential oils in the field of perfumes or flavors.

It is known that the key substances of the essential oils are, for example, (+)-cis-alpha-irone, (-)-trans-alpha-irone, (+)-beta-irone, and (+)-cis-gamma-irone represented by the following formulae present as a mixture.

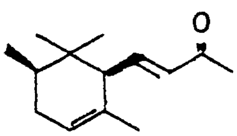

(+)-cis-alpha-irone

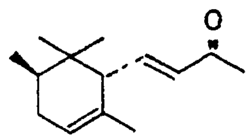

(-)-trans-alpha-irone

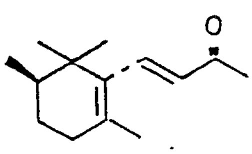

(+)-beta-irone

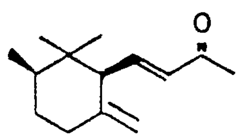

(+)-cis-gamma-irone

On the other hand, (±)-trans-gamma-irone, (±)-cis-gamma-irone and (+)-trans-gamma-irone of the following formulae, for example, are known as synthesized gamma-isomers although they do not exist in the aforesaaid essential oils.

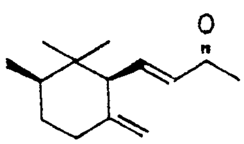

(±)-cis-gamma-irone

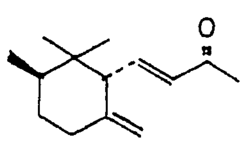

(+)-trans-gamma-irone
(±)-trans-gamma-irone

It is known that among the compounds known as the key substances of the natural essential oils, (+)-cis-gamma-irone has a fragrance characteristic of the above essential oils, but no case has been known in

which it was successfully synthesized.

No example of the presence of the (±)-cis-gamma-irone represented by formula (B) in this invention has been known. The compound (B) is a substance which is described, for example, in Agric. Biol. Chem., 47 (3), 581-586, 1983. According to this literature reference, it is known that in accordance with the following flow chart, (±)-cis-gamma-irone of formula (B) can be formed in the form of a mixture of (±)-trans-gamma-irone of formula (A) and (±)-cis-gamma-irone of formula (B).

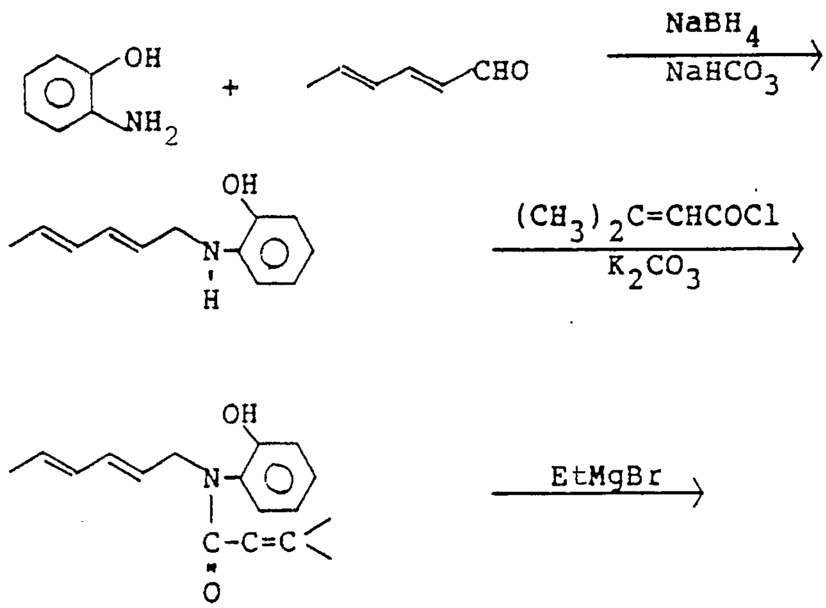

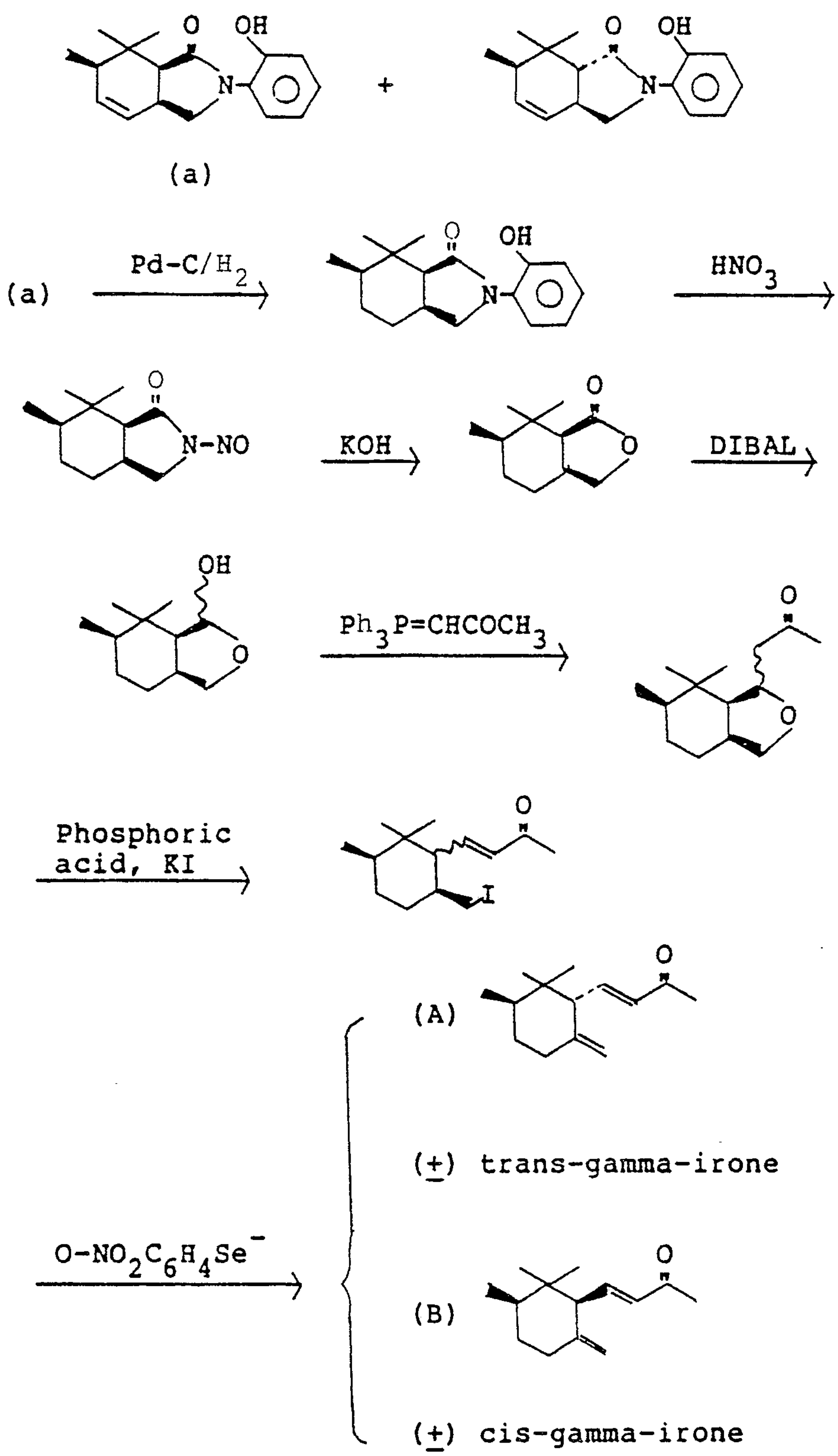


However, according to this proposal, a mixture of the trans-isomer and the cis-isomer is formed, and the cis-isomer cannot be selectively formed. In addition, it has the disadvantage that the proportion of the cis-isomer formed is very low as shown by the trans/cis ratio of 9:1. Moreover, as the above flow chart indicates, it requires many very complex steps and expensive materials. The yield is low and it is not at all suitable for industrial practice. Thus, this prior proposal has many defects and disadvantages.

Furthermore, this reference does not at all refer to the isolation of the (±)-cis-gamma-irone, and the presence of the compound (B) was determined only by instrumental analysis of the resulting mixture of (A) and (B). Naturally, the reference neither discloses nor suggests the characteristic of the odor of the compound of formula (B), its utility and the method of isolating it.

**Disclosure of the Invention**

The present inventors made investigations in order to provide a process for synthesizing only the (±)-cis-gamma-irone of formula (B) above containing no trans-isomer and to elucidate the fragrance characteristics of the compound of formula (B).

Consequently, the present inventors have found that (±)-cis-gamma-irone of formula (B) alone can be produced industrially advantageously through quite a new synthesis route not involving the formation of (±)-trans-gamma-irone of formula (A). It has been found particularly that (±)-cis-gamma-irone represented by the following formula (B)

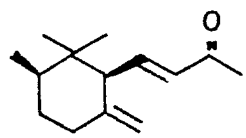

..... (B)

can be synthesized selectively and easily in good yields without the formation of the by-product trans-isomer by contacting (±)-cis-3-(2,2,3-trimethyl-6-methylenecyclohexyl)-acrylonitrile represented by the following formula (4)

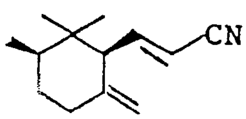

..... (4)

with methyllithium. The compound of formula (4) is not described in the prior literature and can be synthesized easily in good yields in three steps from cis-2,2,3-trimethyl-6-methylene-cyclohexylacetaldehyde of formula (1) given hereinafter (the novel compound synthesized for the first time by the present inventors) which can be provided easily at low cost from 3,3,4-trimethyl-1-cyclohexenylmethanol of formula (a) given hereinafter.

Investigations of the present inventors have shown that the compound of formula (B) has a particularly superior fragrance to those of, for example, (±)-cis-alpha-irone, (±)-trans-alpha-irone, (±)-beta-irone and (±)-trans-gamma irone previously known, and its fragrance is long-lasting. It has also been found that the compound of formula (B) is a unique compound noteworthy in the field of perfumes or flavors which has a mellow fragrance and flavor comparable to, or even superior to, natural orris oil and also its fragrance and flavor are long-lasting. Another advantage is that this compound can be produced in high purity at low cost with industrial ease, and is very useful as an agent for imparting or modifying a long-lasting fragrance or flavor. It has also been found that the compound of formual (B) is a noteworthy useful compound which is in good accord with various natural and synthetic perfumes and flavors, and when blended with fragrance compositions of woody, floral, green, fruit, musk and citrus notes, can be used as an agent for imparting an excellent long-lasting fragrance or flavor or as an agent for modifying such fragrance or flavor.

It is an object of this invention therefore to provide a process for producing (±)-cis-gamma-irone without the formation of the trans-isomer, and a long-lasting fragrance or flavor imparting or modifying agent comprising the compound of formula (B) as an active ingredient.

The above and other ojbects and advantages of this invention will become more apparent from the following description.

The (±)-cis-gamma irone of formula (B) in accordance with this invention can be produced industrially advantageously, for example by the procedure shown in the following reaction scheme.

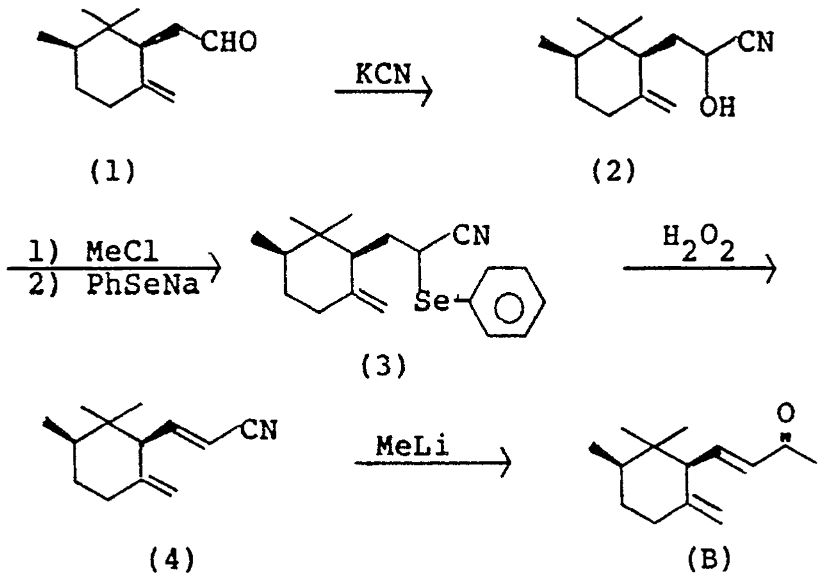


(1) (2) (3) (4) (B)

The production of the compound of formula (B) will be described in more detail by taking the above embodiment as an example.

(±)-cis-3-(2,2,3-Trimethyl-6-methylenecyclohexyl)-2-hydroxypropionitrile of formula (2) above can be easily carried out by contacting (±)-cis-2,2,3-trimethyl-6-methylene-cyclohexylacetaldehyde of formula (1) above with potassium cyanide in the presence of acetic acid, preferably in an organic solvent.

The reaction can be carried out preferably at a temperature of, for example, about 30 to about 150° C for a period of, for example, about 1 to about 5 hours. Specific examples of the organic solvent used in the reaction are alcohols such as methanol, ethanol or propanol. There is no particular restriction on the amount of the organic solvent used, and it can be properly selected. For example, it is preferably about 1 to about 10 times the weight of the compound of formula (1). The amount of potassium cyanide used in the reaction is, for example, about 1 to about 30 moles per mole of the compound of formula (1). The amount of acetic acid used is, for example, about 1 to about 10 moles per mole of potassium cyanide. After the reaction, the reaction mixture is extracted with an organic solvent such as ether, neutralizd with a suitable alkali, and washed with water to remove the solvent. The residue is purified by such means as distillation or column chromatography. Thus, the compound of formula (2) can be easily produced.

(±)-cis-3-(2,2,3-Trimethyl-6-methylenecyclohexyl)-2-phenylselenyl propionitrile of formula (3) can be easily synthesized from the compound (2) which can be obtained as above, by contacting the compound of formula (2) with mesyl chloride in the presence of a basic catalyst to form a mesylate of the compound of formula (2), and then contacting the mesylate compound with sodium selenophenoxide prepared in advance.

The basic catalyst used in the above reaction may preferably be an organic base such as pyridine, piperidine, triethylamine or pyrrolidine. The preferred amount of the basic catalyst used is, for example, about 1 to about 5 moles per mole of mesyl chloride. The reaction can be carried out at a temperature of, for example about -5° C to about 50° C for a period of, for example, about 1 to about 5 hours. The amount of the sodium phenylselenide to be contacted with the mesylate compound of the compound of formula (2) is, for example, about 1 to about 10 moles per mole of the mesylate compound. The reaction is preferably carried out in an organic solvent such as ethanol or methanol. The reaction temperature and time can be selected properly depending upon the type of the solvent used. For example, temperatures of about -5° C to about 100° C and reaction periods of about 1 to about 10 hours may be cited. After the reaction, the reaction mixture is extracted with an organic solvent such as ether and washed with water. The solvent is distilled off, and the residue is purified by using such means as column chromatography or distillation. Thus, the compound of formula (3) can be easily synthesized.

(±)-cis-3-(2,2,3-Trimethyl-6-methylcyclohexyl)acrylonitrileof formula (4) can be easily synthesized from the compound of formula (3) which can be obtained as above, by, for example, contacting the compound of formula (3) with hydrogen peroxide in the presence of a base.

The reaction is carried out, preferably in an organic solvent, at a temperature of, for example, about 0° C to about 50° C and a reaction time of, for example, about 10 to about 120 minutes. Specific examples of the organic solvent used in the reaction are dichloromethane, trichloromethane and carbon tetrachloride.

Preferred examples of the base used are pyridine, piperidine and triethylamine. The amount of aqueous hydrogen peroxide used is, for example, about 1 to about 10 moles per mole of the compound of formula (3). After the reaction, the reaction mixture is extracted with an organic solvent such as ether and washed with an aqueous solution of copper sulfate and water, and the solvent is distilled off. Then, by using such means as column chromatography or distillation, the compound of formula (4) can be easily synthesized.

(±)-cis-gamma-Irone of formula (B) can be easily synthesized from the compound of formula (4) which can be obtained as described above, by contacting the compound of formula (4) with methyllithium.

The reaction is carried out preferably in an organic solvent. Such organic solvents as ether and tetrahydrofuran can be preferably utilized. The reaction temperature and time can be properly selected also according to the solvent used, and reaction temperatures of about -60 °C to about 50 °C and reaction periods of about 1 to about 5 hours may be cited. The amount of methyllithium used in the above reaction is, for example, about 1 to about 10 moles per mole of the compound of formula (4) preferably. After the reaction, the product is treated with ammonium chloride, extracted with an organic solvent such as ether, and washed with water. The solvent is distilled off, and the residue is purified by such means as distillation or column chromatography. Thus, the compound of formula (B) can be synthesized easily.

In the present invention, the cis-2,2,3-trimethyl-6-methylene-cyclohexylacetaldehyde of formula (1) can be industrially advantageously produced, for example, by the following procedure schematically shown.

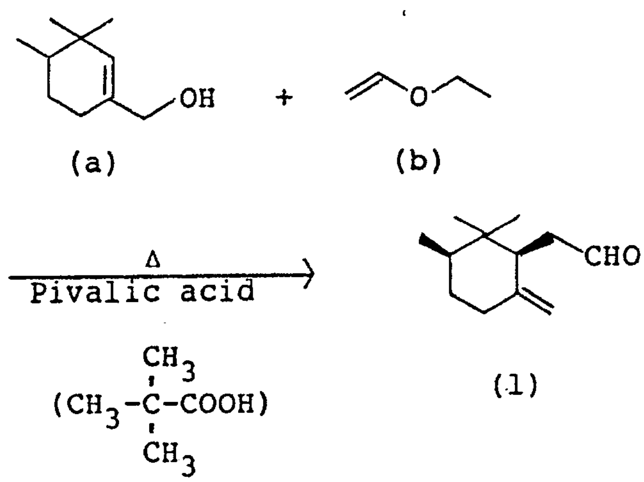


(a) (b) (1)

In the above embodiment, the compound of formula (1) can be produced from the compound of formula (a) by, for example, reacting the compound of formula (a) with the compound of formula (b) in the presence of pivalic acid in an organic solvent or in the absence of a solvent, preferably under heat. The reaction can be caried out, for example, in a closed vessel at a temperature of, for example about 50 °C to about 300 °C, preferably about 150 to about 250 °C. The reaction time can also be suitably selected, and the compound of formula (1) can be easily synthesized by carrying out the reaction for a period of, for example, about 1 to about 5 hours. The above reaction is carried out preferably in an inert gas such as nitrogen gas.

In the above reaction, the amount of the alkyl vinyl ether of formula (b) can be properly chosen, and for example, it is about 1 to about 10 moles, preferably about 1.2 to about 2 moles, per mole of the compound of formula (a). Specific examples of the compound of formula (b) other than ethyl vinyl ether are methyl vinyl ether, ethyl vinyl ether, propyl vinyl ether, isopropyl vinyl ether, n-butyl vinyl ether, isobutyl vinyl ether and n-amyl vinyl ether. Pivalic acid is a compound easily available on the market. Its amount is, for example, about 0.1 to about 50% by weight, preferably about 10 to about 30% by weight, based on the compound of formula (a).

Where the above reaction is carried out in the presence of an organic solvent, the amount of the organic solvent is, for example, about 10 to about 500% by weight, preferably about 50 to about 200% by weight, based on the compound of formula (a). Specific examples of such organic solvents include toluene, xylene, benzene, petroleum ether, pentane, hexane, ether, tetrahydrofuran, dioxane and diglyme.

The reaction product consists of the cis-isomer and the trans-isomer in a ratio of about 30:70. After the reaction, the reaction product is washed with, for example, an aqueous solution of sodium hydrogen carbonate and the solvent is distilled off. Distillation of the residue under reduced pressure led to the separation of the cis-isomer from the trans-isomer, and the cis-2,2,3-trimethyl-cyclohexylacetaldehyde of formula (1) can be obtained easily in a high yield.

The cis-2,2,3-Trimethyl-6-methylene-cyclohexylacetaldehyde of formula (1), (±)-cis-3-(2,2,3-trimethyl-6-methylcyclohexyl)-2-hydroxypropionitrile of formula (2) (±)-cis-3-(2,2,3-trimethyl-6-methylenecyclohexyl)-2-phenylselenyl propionitrile of formula (3) and (±)-cis-3-(2,2,3-trimethyl-6-methylenecyclohexyl)-acrylonitrile of formula (4) are novel compounds not described in the prior literature, and useful materials for the compound of formula (B) of this invention. In addition, the compound of formula (1) has a sweet, soft fruit-like fragrance, and the compounds of formula (2) and (4) have a long-lasting floral or citrus fragrance or flavor. They well harmonize with various synthetic and natural perfumes and are by themselves useful compounds which can be blended with various compounded fragrance compositions as a long-lasting fragrance or flavor imparting or modifying agent.

It has been found, for example, that (±)-cis-gamma-irone of formula (B) that can be synthesized as above is very useful as a long-lasting fragrance or flavor imparting or modifying agent. The compound of formula (B) has a natural orris oil-like fragrance or flavor and excellent long-lasting. It is mild and natural and has depth and richness, and gives a long-lasting and unique fragrance or flavor as a fragrance or flavor component of various foods, drinks, cosmetics, health-keeping goods, hygienic goods and medicines.

More specificatlly, when (±)-cis-gamma-irone of formula (B) is added to at least one synthetic aromatic chemicals, a novel, long-lasting fragrance or flavor imparting or modifying agent can be prepared. For example, when it is mixed with synthetic essential oils such as bergamot oil, lemon oil, geranium oil, mandarin oil or lavender oil, novel, long-lasting fragrance or flavor imparting or modifying agents having mildness and body inherent to natural essential oils can be prepared. They also harmonize well with natural essential oils such as orange oil, lime oil, grapefruit oil, oakmoss oil, citronella oil, vetiver oil, cinnamon oil, patchouli oil, thyme oil, clove oil, bergamot oil and rose oil, and novel, long-lasting fragrance or flavor imparting or modifying agents emphasizing the characteristics of these essential oils can be prepared.

When the irone of formula (B) is blended with a compounded perfume comprising two or more natural perfumed or synthetic aromatic chemicals, for example a fragrance composition of a single or mixed floral bouquets of rose, jasmine, lilac, osmanthus, carnation or lily of the vally, or modern floral bouquets or Oriental bouquets further having a green note, a spicy note or an aldehyde note, or a flavoring composition of a strawberry, lemon, orange, grapefruit, apple or pinapple flavor, novel fragrance or fravor imparting or modifying agents which are mild and natural and have depth and richness and enhanced long-lasting property can be obtained. The amount of the (±)-cis-gamma-irone of formula (B) to be added varies depending upon the purpose of addition and the type of the fragrance or flavor imparting or modifying agent to which it is added, and is, for example, about 0.001 to about 30% based on the entire composition.

Thus, according to this invention, there can be provided foods and drinks (including articles of taste) characterized by containing (±)-cis-gamma-irone of formula (B) as a fragrance component, cosmetics containing the (±)-cis-gamma-irone of formula (B) as a fragrance component, and health-keeping or hygienic articles or medicines characterized by containing (±)-cis-gamma-irone as a fragrance or flavor component, by utilizing a long-lasting fragrance or flavor imparting agent or modifier or fortifier comprising (±)-cis-gamma-irone of formula (B) as an active ingredient.

For example, (±)-cis-gamma-irone can be incorporated in a suitable amount sufficient to impart its unique fragrance or flavor to various foods and drinks including, for example, drinks such as fruit juices, fruit liquors, milk and milk-derived drinks and carbonated drinks; cold confectionary such as icecream, sherbet and ice candy; aricles of taste such as cakes, jams, chewing gums, bread, coffee, cocoa, black tea and green tea; soups of Western and Oriental types; flavorings, seasonings, various instant foods and drinks, and various snacks. There can also be provided cosmetics obtained by incorporating the irone of formula (B) in a suitable amount sufficient to impart its unique fragrance in shampoos, hair-creams, pomade, and other hair-treating cosmetic bases, and cosmetic bases or cosmetic detergent bases including facial powder and lip-sticks. There can also be provided health-keeping or hygienic aritcles and medicines by incorporating a suitable amount, sufficient to impart its unique flavor, of the irone of formula (B) into health-keeping and hygienic detergents such as washing detergents, disinfecting detergents, odor-controlling detergents and indoor fragrance agents, various health-keeping and hygienic articles such as toothpastes, tissue paper and toilet paper; and health-keeping or hygienic articles and medicines such as corrigents and flavoring agents.

As exemplified extensively hereinabove, the present invention can provide a perfume or flavor composition comprising a perfume or flavor or a perfumed or flavored product and a perfuming or flavoring amount of (±)-cis-gamma-irone represented by formula (B) below

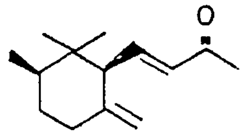

.....(B)

as a component for imparting, modifying or enhancing odor-iferous properties. The perfuming or flavoring amount is, for example, about 0.001 to about 30% by weight based on the weight of the perfume or flavor composition.

According to one embodiment of this invention, a perfume composition comprising about 5 to about 50% by weight of (±)-cis-gamma-irone of formula (B) and about 50 to about 95% by weight of (±)-trans-gamma-irone can be provided.

According to this invention, there is also provided a method of imparting odoriferous properties to a perfume or flavor or a perfumed or flavored product, or modifying or enhancing the odoriferous properties of such a perfume or flavor or such a perfumed or flavored product, which comprises a step of incorporating (±)-cis-gamma-irone of formula (B) in the perfume or flavor or the perfumed or flavored product.

According to this invention, there can also be provided processes for producing (±)-cis-gamma-irone of formula (B) as shown below. According to these processes, the compound of formula (B) can be produced industraiIly advanageously by a shortened process and an easy operation from an inexpensive compound as compared with the prior art.

1. A process for producing (±)-cis-gamma-irone represented by the following formula (B)

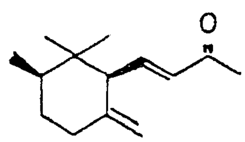

.....(B)

which comprises contacting (±)-cis-3-(2,2,3-trimethyl-6-methylenecyclohexyl)-acrylonitirle represented by the fcllowing formula (4):

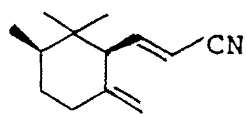

.....(4)

2. A process for producing (±)-cis-gamma-irone represented by the following formula (B)

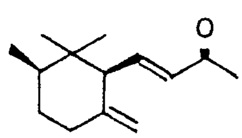

.....(B)

set forth in claim 1, which comprises contacting (±)-cis-2,2,3-trimethyl-6-methylenecyclohexyl acetaldehyde represented by the following formula (1)

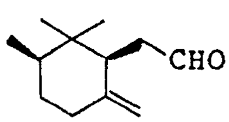

.....(1)

with potassium cyanide in the presence of acetic acid to form (±)-cis-3-(2,2,3-trimethyl-6-methylenecyclohexyl)-2-hydroxypropionitrile represented by the following formula (2)

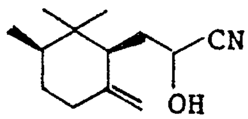

.....(2)

contacting the resulting compound of formula (2) with mesyl chloride (MsCl) in the presence of a base, then contacting the product with sodium phenylselenide (PhSENa) to form (±)-cis-3-(2,2,3-trimethyl-6-methylenecyclohexyl)-2-phenylselenyl propionitrile represented by the following formula (3).

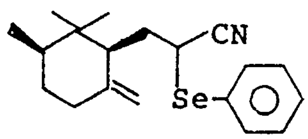

.....(3)

Then contacting the compound of formula (3) with hydrogen peoxide in the presence of a base to form (±)-cis-3-(2,2,3-trimethyl-6-methylenecyclohexyl)-acrylonitrile represented by the following formula (4)

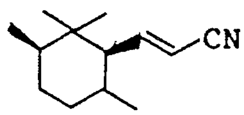

.....(4)

and thereafter contacting the compound of formula (4) with methyllithium.

**Best modefor practicing the invention**

The following Examples illustrate more specifically several embodiments including the best mode of practice in the production and use of the compound of this invention represented by formula (B).

Example 1

Synthesis of cis-2,2,3-trimethyl-6-methylene-cyclohexylacetaldehyde [formula (1)]:-

A 100 ml pressure autoclave was charged with 20 g of 3,3,4-trimethyl-1-cyclohexenylmethanol, 30 g of ethyl vinyl ether and 6 g of pivalic acid. The temperature was raised to 220° C, and they were reacted for 3 hours. After the reaction, the reaction mixture was washed with a saturated aqueous solution of sodium hydrogen carbonate and washed with water to obtain a crude product (cis:trans = -30:70). The crude product was distilled under reduced pressure to give 3.1 g (yield 13%) of cis-2,2,3-trimethyl-6-methylene-cyclohexylacetaldehyde.

IR $\nu$(liquid film): 1725, 1645, 895 $cm^{-1}$

NMR ($CDCl_3$) $\delta$:

0.58 (3H, s), 0.87 (3H, d, J = 5Hz), 0.99 (3H, s), 2.51 (2H, d, J = 2Hz), 4.42 (1H, s), 4.83 (1H, s), 9.64 (1H, t, J = 2Hz).

Example 2

Synthesis of (±)-cis-3-(2,2,3-trimethyl-6-methylene-1-cyclohexyl)-2-hydroxypropionitrile:-

Acetic acid (6.6 g; 0.11 mole) and 4.4 g (68 millimoles) of potassium cyanide were added at 0° C to a solution of 2.4 g (13.4 millimoles) of (±)-cis-2,2,3-trimethyl-cyclohexylacetaldehyde of formula (1) in 40 ml of ethanol. The mixture was stirred at 0° C for 3 hours. The reaction product was extracted with ether. The ethereal layer was washed with water, an aqueous solution of sodium hydrogen carbonate and further with water until the washings became neutral. Finally, the product was washed with a saturated aqueous solution

of sodium chloride and dried over magnesium sulfate. The solvent was removed to give 2.4 g of an oil of formula (2). Yield 89%.

IR ν(liquid film): 3450, 3060, 1640, 895, 2240 $cm^{-1}$.

Example 3

Synthesis of (±)-cis-3-(2,2,3-trimethyl-6-methylene-1-cyclohexyl)-2-phenylselenyl propionitrile of formula (3):-

Mesyl chloride (2.4 ml; 30 milimoles) was added at -4° C to -3° C to a solution of 2.06 g (10 millimoles) of (±)-cis-3-(2,2,3-trimethyl-6-methylene-1-cyclohexyl)-2-hydroxypropionitrile of formula (2) in 20 ml of pyridine, and under an argon stream, the mixture was stirred at the same temperature for 5 hours. The reaction mixture was added to water, and extracted with ether. The ethereal layer was washed with a 10% aqueous solution of copper sulfate, water and a saturated aqueous solution of sodium salt, and dried over anhydrous sodium sulfate. The solvent was distilled off to give 2.9 g of a mesylate compound as an oil. Separately, 0.54 g (14 millimoles) of sodium borohydride was added at room temperature to a solution of 2.2 g (7 millimoles) of diphenyl diselenide in 12 ml of ethanol under an argon stream, and the mixture was stirred. When the evolution of gas ceased and the solution became colorless and transparent, a solution of 2.9 g of the crude mesylate in 11 ml of ethanol as added at -6° C to -7° C, and the mixture wass stirred at room temperature for 2 hours. The reaction product was added to water and extracted with ether. The ethereal layer was washed with water and a saturated aqueous solution of sodium chloride, and then dried over anhydrous sodium sulfate. The solvent was distilled off to give 2.64 g of the compound of formula (3) as an oil. Yield 77%.

Example 4

Synthesis of (±)-cis-3-(2,2,3-trimethyl-6-methylene-1-cyclohexyl)-2-acrylonitrile:-

To 1.5 ml of pyridine and a solution of 2.8 g (4 millimoles) of (±)-cis-3-(2,2,3-trimethyl-6-methylene-1-cyclohexyl)-2-phenylselenyl propionitrile of formula (3) in 15 ml of methylene chloride was added 18 ml (80 millimoles) of 15% hydrogen peroxide at 8 to 10° C, and the mixture was stirred at the same temperature for 2 hours. The reaction product was extracted with ether. The ethereal layer was washed successively with a 10% aqueous solution of copper sulfate, water and a saturated aqueous solution of sodium chloride, and then dried over anhydrous sodium sulfate. The solvent was distilled to give 1.14 g of the compound of formula (4). Yield 75%.

IR ν(liquid film): 3075, 2225, 1640, 1630, 895 $cm^{-1}$.

$^1H$-NMR ($CDCl_3$) δ:

0.69 (3H, s), 0.85 (3H, d, J=5Hz), 0.89 (3H, s), 1.0-2.4 (5H, m), 2.55 (1H, d, J=10Hz), 4.40 (1H, s), 4.81 (1H, s), 5.33 (1H, d, J=16Hz), 6.84 (1H, d-d, J=16 and 10Hz).

Example 5

Synthesis of (±)-cis-gamma-irone of formula (B):-

1.5M methyllithium (4.2 ml; 6.2 millimoles) was added at -65° C to -55° C to a solution of 920 mg (5 millimoles) of (±)-cis-3-(2,2,3-trimethyl-6-methylene-1-cyclohexyl)-2-acrylonitrile in 40 ml of ether, and the mixture was stirred at the same temperature for 3 hours and then at 0 °C for 1 hour. The reaction mixture was added to ice water + aqueous ammonium chloride solution, and then extracted with ether. The ethereal layer was washed with water and a saturated aqueous solution of sodium chloride, and dried over magnesium sulfate. The ether was distilled off to give 500 mg of the compound of formula (B). Yield 50%.

MS: m/e 206 ($M^+$)

IR ν(liquid film): 3070, 1680, 1640, 1625, 895.

$^1H$-NMR ($CDCl_3$) δ:

0.71 (3H, s), 0.84 (3H, d, J=5Hz), 0.86 (3H, s), 1.0-1.9 (5H, m) , 2.26 (3H, s), 2.55 (1H, d, J=10Hz), 4.44 (1H, s), 4.79 (1H, s), 6.08 (1H, d, J=16Hz), 6.97 (1H, d-d, J=16Hz and 10Hz).

Example 6

A compounded perfume composition of the bouquet type was prepared by mixing the following components (in parts by weight).

| | |
|---|---|
| Phenethyl alcohol | 180 |
| Linalyl acetate | 30 |
| Bergamot oil | 40 |
| Geranium oil | 50 |
| Benzyl acetate | 60 |
| Heliotropin | 80 |
| Geraniol | 110 |
| Lavender oil | 20 |
| beta-Ionone | 100 |
| Amyl salicylate | 45 |
| Terpinyl acetate | 135 |
| Cedar wood oil | 100 |
| Citronellol | 50 |
| | 1000 |

By adding 20 g of (±)-cis-gamma-irone to 980 g of the above composition, a novel perfume composition of a bouquet note was obtained which was of more natural floral note having excellent long-lasting characteristics.

Twenty grams of (±)-cis-alpha-irone was added instead of (±)-cis-gamma-irone to 980 g of the above composition to form a blended perfume composition. Compounded perfume compositions were also prepared in the same way by using (±)-trans-alpha-irone, (±)-beta-irone and (+)-trans-gamma-irone. These perfume compositions were compared with the blended perfume composition containing the compound of this invention by a panel of 10 specialists. As a result, all the 10 panelists reported that the compounded perfume composition containing the compound of this invention had far superior long-lasting fragrance.

Example 7

A rose-type compounded perfume composition was prepared by mixing the following components (parts by wight).

| | |
|---|---|
| Phenethyl alcohol | 200 |
| Geraniol | 50 |
| Coumarin | 20 |
| Heliotropin | 10 |
| Citronellol | 100 |
| Nerol | 30 |
| Hydroxycitronellal | 20 |
| Methylphenylcarbinyl acetate | 5 |
| Geranium oil | 10 |
| Linalool | 30 |
| Benzyl acetate | 35 |
| Benzyl alcohol | 20 |
| Rosephenone | 10 |
| Rhodinol | 280 |
| Rose oil | 10 |
| beta-Ionone | 50 |
| Benzyl salicylate | 40 |
| Cyclopentadecanolide | 30 |
| Guaiac wood oil | 50 |
| | 1000 |

By mixing 960 g of the above composition with 40 g of (±)-cis-gamma-irone, a novel rose perfume composition which was natural, fresh and long-lasting was obtained.

A compounded perfume composition was prepared by adding 40 g of (±)-cis-gamma-irone to 960 g of the composition.

Compounded perfume compositions were also prepared by adding (±)-trans-alpha-irone, (±)-beta-irone and (+)-trans-gamma-irone. These compositions were compared with the compounded perfume composition containing the irone in accordance with this invention by a panel of ten specialists. All of the 10 panelists reported that the compounded perfume composition containing the compound of this invention had much superior fragrance of long-lasting characteristics.

Example 8

The following components (parts by weight) were mixed to prepare a floral-type compounded perfume composition.

| | |
|---|---|
| Cyclopentadecanolide | 50 |
| Coumarin | 10 |
| Heliotropin | 30 |
| Ylang-ylang oil | 80 |
| Methylionone | 100 |
| Anisaldehyde | 20 |
| Neroli oil | 30 |
| Hydroxycitronellal | 50 |
| Linalool | 70 |
| iso-Butyl salicylate | 110 |
| Amyl salicylatte | 140 |
| 5-Cyclohexadecenone | 20 |
| Oakmoss absolute | 3 |
| Vanillin | 7 |
| Phenyl acetaldehyde | 20 |
| Phenethyl alcohol | 200 |
| Benzyl acetate | 60 |
| | 1000 |

By adding 90 g of (±)-cis-gamma-irone to 910 g of the above composition, a novel compounded perfume composition having a long-lasting and enhanced fresh and more natural floral note was obtained.

A compounded perfume composition was prepared by adding 90 g of (±)-cis-alpha-irone instead of (±)-cis-gamma-irone. Likewise, compounded perfume compositions were prepared by adding (±)-trans-alpha-irone, (±)-beta-irone and (+)-trans-gamma-irone. These compounded perfume compositions were compared with the compounded perfume composition containing the compound of this invention by a panel of 10 specialists. As a result, all of the 10 panelists reported that the compounded perfume composition containing the compound of this invention had much superior fragrance of long-lasting characteristics.

Example 9

An apricot fragrance and flavor composition was prepared by mixing the following components.

| | |
|---|---|
| Allyl cyclohexyl caproate | 0.2 |
| Benzaldehyde | 11.5 |
| Amyl acetate | 7.5 |
| Amyl butyate | 7.5 |
| Amyl formate | 10.0 |
| Amyl valerate | 15.0 |
| Cinnamic aldehyde | 0.5 |
| Ethyl acetate | 14.5 |
| Ethyl butyrate | 4.5 |
| Ethyl hexanoate | 10.0 |
| Ethyl valerate | 50.0 |
| Geranium oil | 0.5 |
| iso-Amyl phenyl acetate | 0.1 |
| Benzyl acetate | 9.5 |
| Lemon oil | 5.0 |
| Orange oil | 10.5 |
| Propyl cinnamate | 0.2 |
| Alpha-undecalactone | 213.0 |
| Vanillin | 85.0 |
| Ethanol | 517.0 |
| | 1000.0 |

Ten grams of the (±)-cis-gamma-irone in accordance with this invention was added to 1000 g of the above compositicn, an excellent novel composition having a fragrance and flavor of apricot of excellent long-lasting characteristics was obtained.

Furthermore, 10 g of (±)-cis-alpha-irone was added instead of (±)-cis-gamma-irone to 1000 g of the above composition. Likewise, compounded perfume compositions were prepared by using (±)-trans-alpha-irone, (±)-beta-irone and (+)-trans-gamma-irone. These compounded perfume compositions were compared with the compounded perfume composition containing the ccmpound of this invention by a panel of 10 specialists. All of the 10 panelists reported that the compounded perfume composition containing the compound of this invention had much superior fragrance of long-lasting characteristics.

Example 10

The following components (parts by weight) were mixed to prepare a bouquet-type compounded perfume composition.

| | |
|---|---|
| Phenethyl alcohol | 180 |
| Linalyl acetate | 30 |
| Bergamot oil | 40 |
| Rhodinol | 50 |
| Benzyl acetate | 40 |
| Heliotropin | 80 |
| Geraniol | 110 |
| Rose absolute | 10 |
| Lavender oil | 20 |
| Jasmine absolute | 10 |
| beta-Ionone | 100 |
| Amyl salicylate | 45 |
| Linalyl acetate | 135 |
| Vetiver acetate | 100 |
| Citronellol | 50 |
| | 1000 |

By adding 2 g of (±)-cis-gamma-irone and 18 g of (±)-trans-gamma-irone to 980 g of the above composition, a novel bouquet compounded perfume composition having a more natural floral note and excellent long-lasting characteristics was obtained.

To 980 g of the above composition was added 20 g of (±)-cis-alpha-irone instead of the above (±)-cis-gamma-irone and (±)-trans-gamma-irone to obtain a compounded perfume composition. Likewise, compounded perfume compositions were prepared by using (±)-trans-alpha-irone, (±)-beta-irone and (±)-trans-gamma-irone. These compounded perfume compositions were compared with the compounded perfume composition containing the compound of this invention by a panel of 10 specialists. As a result, nine panelists out of ten reported that the compounded perfume composition containing the compound of this invention had much superior fragrance and long-lasting characteristics.

Compounded pefume compositions were prepared in the same way as above using a mixture of 1 g of (±)-cis-gamma-ironeand 19 g of (±)-trans-gamma-irone and a mixture of 6 g of (±)-cis-gamma-irone and 14 g of (±)-trans-gamma-irone. These compositions were novel bouquet compounded perfume composition having a natural floral note and excellent long-lsting characteristics.

## Industrial utilizabilty

(±)-cis-gamma-Irone can be utilized as an agent for imparting odoriferous properties to a perfume or flavor or a perfumed or flavored product or as an agent for modifying or enhancing these odoriferous properties in a wide range of applications including foods, drinks, cosmetics, health-keeping and hygienic articles, and medicines.

## Claims

1. A perfume or flavor composition comprising a perfume or flavor or a perfumed or flavored product and a perfuming or flavoring amount of (±)-cis-gamma-irone represented by the following formula (B) :

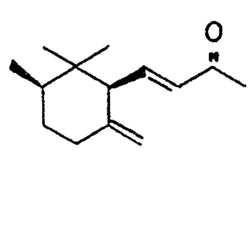

.....(B)

2. The composition set forth in claim 1 which further comprises (±)-trans-gamma-irone in a proportion of about 50 to about 95% by weight as against about 5 to about 50% by weight of (±)-cis-gamma-irone of formula (B).

3. The composition set forth in claim 1 or 2 wherein the content of (±)-cis-gamma-irone of formula (B) is about 0.001 to about 30% by weight based on the weight of the composition.

4. A method of imparting odoriferous properties to a perfume or flavor or a perfumed or flavored product, or modifying or enhancing its odoriferous properties, which comprises a step of blending (±)-cis-gamma-irone represented by the following formula (B)

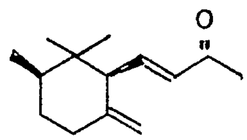

.....(B)

with the perfume or flavor or the product to be perfumed or flavored.

5. The method of claim 4 which further comprises blending about 50 to about 95% by weight of (±)-trans-gamma-irone as against about 5 to about 50% by weight of (±)-cis-gamma-irone of formula (B).

6. A process for producing (±)-cis-gamma-irone represented by the following formula (B)

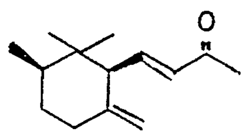

.....(B)

which comprises contacting (±)-cis-3-(2,2,3-trimethyl-6-methylénecyclohexyl)-acrylonitrile represented by the following formua (4)

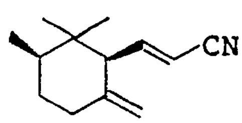

.....(4)

with methyllithium.

**Revendications**

1. Composition de parfum ou d'arôme comprenant un parfum ou arôme ou un produit parfumé ou aromatisé et une quantité à effet parfumant ou aromatisant de (±)-cis-gamma-irone représentée par la formule (B) :

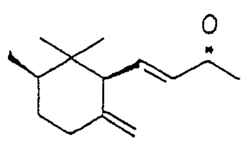

.....(B)

2. Composition selon la revendication 1, qui comprend, de plus, de la (±)-trans-gamma-irone en une proportion d'environ 50 à environ 95 % en poids vis-à-vis d'environ 5 à environ 50 % en poids de (±)-cis-gamma-irone de formule (B).

3. Composition selon la revendication 1 ou 2, dans laquelle la proportion de (±)-cis-gamma-irone de formule (B) est d'environ 0,001 à environ 30 % en poids par rapport au poids de la composition.

4. Procédé pour conférer des propriétés odorifiques à un parfum ou arôme ou à un produit parrumé ou aromatisé, ou pour modifier ou exalter ses propriétés odoriférantes, qui comprend l'étape consistant à incorporer la (±)-cis-gamma-irone représentée par la formule (B) suivante :

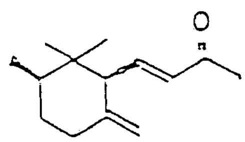

.....(B)

au parfum ou arôme ou au produit à parfumer ou aromatiser.

5. Procédé selon la revendication 4, qui comprend, de plus, l'incorporation d'environ 50 à environ 95 % en poids de (±)-trans-gamma-irone vis-à-vis d'environ 5 à environ 50 % en poids de (±)-cis-gamma-irone de formule (B).

6. Procédé pour produire la (±)-cis-gamma-irone représentée par la formule (B) :

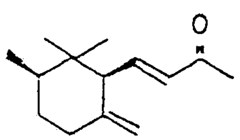

.....(B)

qui consiste à mettre en contact le (±)-cis-3-(2,2,3-triméthyl-6-méthylène-cyclohexyl)acrylonitrile représenté par la formule (4) suivante :

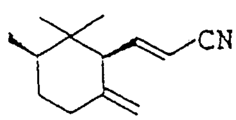

.....(4)

avec du méthyl-lithium.

**Patentansprüche**

1. Parfüm- oder Aromazusammensetzung, dadurch **gekennzeichnet,** daß sie ein Parfüm oder Aroma oder ein parfümiertes oder aromatisiertes Produkt und eine parfümierende oder aromatisierende Menge von (±)-Cis-gamma-iron der folgenden Formel (B):

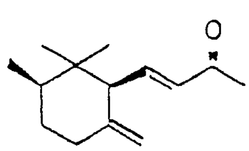

.....(B)

enthält.

2. Zusammensetzung nach Anspruch 1, dadurch **gekennzeichnet,** daß sie weiterhin (±)-Trans-gamma-iron in einem Verhältnis von etwa 50 bis etwa 95 Gew.-% zu etwa 5 bis etwa 50 Gew.-% (±)-Cis-gamma-iron der Formel (B) enthält.

3. Zusammensetzung nach Anspruch 1 oder 2, dadurch **gekennzeichnet,** daß der Gehalt an (±)-Cis-gamma-ironder Formel (B) etwa 0,001 bis etwa 30 Gew.-%, bezogen auf das Gewicht der Zusammensetzung, beträgt.

4. Verfahren, einem Parfüm oder Aroma oder einem parfümierten oder aromatisierten Produkt duftende bzw. riechende Eigenschaften zu verleihen oder dessen duftende bzw. riechende Eigenschaften zu modifizieren oder zu verstärken, dadurch **gekennzeichnet,** daß man (±)-Cis-gamma-iron der folgenden Formel (B)

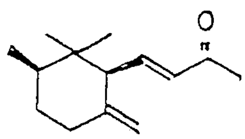

.....(B)

mit dem Parfüm oder Aroma oder dem zu parfümierenden oder aromatisierenden Produkt vermischt.

5. Verfahren nach Anspruch 4, dadurch **gekennzeichnet,** daß man weiter etwa 50 bis etwa 95 Gew.-% (±)-Trans-gamma-iron zu etwa 5 bis etwa 50 Gew.-% (±)-Cis-gamma-iron der Formel (B) vermischt.

6. Verfahren zur Herstellung von (±)-Cis-gamma-iron der folgenden Formel (B)

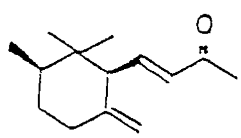

.....(B)

dadurch **gekennzeichnet,** daß man (±)-Cis-3-(2,2,3-trimethyl-6-methylencyclohexyl)-acrylonitril der folgenden Formel (4)

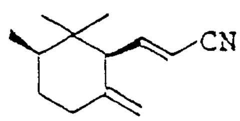

.....(4)

mit Methyllithium in Kontakt bringt.